Europäisches Patentamt

European Patent Office

Office européen des brevets

(11) Publication number: **0 178 122 B1**

## EUROPEAN PATENT SPECIFICATION

(45) Date of publication of patent specification: 05.06.91

(51) Int. Cl.⁵· **A61K 31/54**, A61K 31 55,
//(A61K31/54,31:505,31:44,
31:335),(A61K31/55,31:54),
(A61K31/54,31:165)

(21) Application number: 85307056.3

(22) Date of filing: 02.10.85

(54) Improved piroxicam-containing antiinflammatory compositions.

(30) Priority: 11.10.84 US 659602
28.01.85 US 695590
28.01.85 US 695518

(43) Date of publication of application:
16.04.86 Bulletin 86/16

(45) Publication of the grant of the patent:
05.06.91 Bulletin 91/23

(84) Designated Contracting States:
AT BE CH DE FR GB IT LI LU NL SE

(56) References cited:

UNLISTED DRUGS, vol. 36, no. 6, June 1984,
page 104, Chatham, NJ, US; "Algio pironal"

(73) Proprietor: PFIZER INC.
235 East 42nd Street
New York, N.Y. 10017(US)

(72) Inventor: Crawford, Thomas Charles
23, Philip Lane
Ledyard Connecticut(US)
Inventor: Larson, David Lee
12, Green Valley Lakes Road
East Lyme Connecticut(US)
Inventor: Maciejko, James John
15, Mystic River Square
Mystic Connecticut(US)
Inventor: Keely, Stanley Leroy
7, Cranwood Way
Ledyard Connecticut(US)
Inventor: Lombardino, Joseph George
13, Laurel Hill Drive
Niantic Connecticut(US)

(74) Representative: Wood, David John et al
PFIZER LIMITED, Ramsgate Road
Sandwich, Kent CT13 9NJ(GB)

**Description**

The present invention is concerned with improved antiinflammatory compositions which employ antiinflammatory piroxicam, or a pharmaceutically acceptable salt thereof (particularly the ethanolamine salt), in combination with bronchodilator pirbuterol, minor tranquilizer diazepam, or antihypertensive trimazosin. The generic names used here and elsewhere herein are from the USAN and the USP Dictionary of Drug Names, 1961-1981, Griffiths et al., ed., U.S. Pharmacopeial Convention Inc., Rockville, Md., 1984, have subsequently been assigned and published as official USAN names, and/or appear in the Merck Index 10th Edition.

Gastrointestinal irritation, including ulcers, is a side effect commonly associated, to one degree or another, with antiinflammatory agents. In many cases, individuals requiring such antiinflammatory treatment are precluded from enjoying the benefits thereof because of their susceptibility to such side effects. The combination of piroxicam with one or another of the medicinal agents defined above permits desirable antiinflammatory therapy while preventing or ameliorating said gastrointestinal irritation or ulcers.

Acetaminophen has been previously reported to reduce the ulcerogenicity of aspirin [Sugers et al., J. Pharm. Pharmacol. 30, 84 (1978); ibid. 31, 840 (1979); and Adv. Prost. Thromb. Res. 8, 1547 (1980)], or of acidified aspirin [Konturek et al., Gut 23, 536 (1982)]. However, indomethacin reversed the protective effect of acetaminophen when given with acidified aspirin (loc. cit.). In later studies, it was reported that acetaminophen reduced the ulcerogenicity of indomethacin and aspirin, but not of phenylbutazone or glafenine, and of ibuprofen only at the highest dose (800 mg/kg) of the latter compound [van Kolfschoten et al., Agents Actions 12, 247 (1982); Toxicology Applied Pharm. 69, 37 (1983)]. Acetaminophen in combination with ketoprofen and other particular antiinflammatory agents has been reported to provide an analgesic effect which is greater than a simple additive effect (U.S. Patents 4,233,313 to 4,233,317; 4,234,601; 4,207,340; and 4,242,353). There are no known prior reports concerning combination of acetaminophen with piroxicam or any other oxicam for any purpose.

Bronchodilators salbutamol (albuterol), phenylephrine and isoproterenol, but not propranolol have been reported to inhibit formation of indomethacin-induced ulcers in animals [Fielding et al., Eur. Surg. Res. 9, 252 (1977); Kasuya et al., Japan J. Pharmacol. 29, 670 (1979)]. In another study, administration of isoproterenol to a chambered section of a dog's fundus reduced or prevented aspirin-induced tissue damage [McGreevy et al., Surg. Forum 28, 357 (1977)]. There are no known prior reports concerning the effect of bronchodilator pirbuterol on antiinflammatory agents.

Antidepressant doxepin has also been reported to have gastric antisecretory activity and to be as effective as cimetidine in the treatment of duodenal ulcers in humans [Hoff et al., Curr. Med. Res. Opin. vol. 6, supplement 9, page 36 (1980); Scand. J. Gastroent. 16, 1041 (1981)]. It has also been reported that doxepin shows antiulcer and antisecretory activity in rats and dogs; and that it significantly reduced the ulcerogenic potential of indomethacin, diclofenac and aspirin in water-immersion restraint-stressed rats [Leitold et al. Arch. Pharmacol. 316 (supplement), R50, abstract 199 (1981); Leitold et al., Advances in Experimental Ulcer, Umehara and Ito, editors, ICEU, Tokyo pp. 27-36 (1982); Arzneim-Forsch/Drug Res. 34, 468 (1984).

Major tranquilizer and antipsychotic chlorpromazine has been reported to reduce indomethacin-induced gastric ulcers in rats [Kasuya et al. loc. cit. (1979)]. Most recently, it has been reported that the immunoregulator levamisole reduced both piroxicam- and indomethacin-induced ulcers in rats [Evangelista et al., J. Pharm. Pharmacol. 36, 270 (1984)]. However, there are no known prior reports concerning the use of an antihypertensive agent such as trimazosin or a minor tranquilizer such as diazepam in reducing gastric side-effects induced by nonsteroidal antiinflammatory agents.

Phospholipids have also been reported to reduce gastrointestinal distress (damage to the mucous membrane, gastric ulcer formation) when combined with nonsteroidal antiinflammatory agents, particularly phenylacetic or phenylpropionic acid derivatives such as ibuprofen, naproxen, diclofenac and flurbiprofen [Ghyczy et al., U.S. Patent 4,369,182 (1983)], as have antiulcer pirenzepine [Leitold et al., Therapiewoche 27, pp. 1532-1548 (1977); German Patent Application 2,708,520] and histamine-$H_2$ antagonist (gastric acid antisecretory, antiulcer) compounds such as ranitidine, cimetidine and 1-methyl-5-[[3-[3-(1-piperidinylmethyl)phenoxy]-propylamino]]-1H-1,2,4-triazole-3-methanol, which have been previously combined with indomethacin and other antiinflammatory agents for this purpose. See, for example, U.K. Patent Applications 2,105,193 and 2,105,588; and Lovelace, U.S. Patent 4,230,717.

Concurrently filed European patent application EP-A-0178124 describes improved antiinflammatory salts of piroxicam, including those with trimazosin, doxepin, N-demethyldoxepin, pyridoxine, isoproterenol and pirbuterol.

The present invention concerns an improved antiinflammatory composition which comprises an antiinflammatory amount of piroxicam, or a pharmaceutically acceptable salt thereof (particularly the ethanolamine salt), in combination with a piroxicam-induced gastric irritation and ulcer-inhibiting amount of a compound selected from pirbuterol, diazepam and trimazosin and the pharmaceutically acceptable salts thereof (except diazepam).

The antiinflammatory agent of the present invention, piroxicam, is known. For example, The Merck Index 10th Ed., 1983 contains a monograph concerning piroxicam (no. 7378), as does the Physicians' Desk Reference (PDR), 38th Ed., pp. 1556-1557 (1984). The preferred ethanolamine salt of piroxicam is specifically disclosed in U.S. Patent 4,434,164.

The compounds of the present invention which we have found to inhibit piroxicam-induced gastric irritation and ulcers are also known compounds. Pirbuterol is a bronchodilator marketed or to be marketed around the world in the form of its dihydrochloride and monoacetate salts. See The Merck Index 10th Ed., monograph no. 7364. Its early synthesis and utility as a bronchodilator is disclosed in U.S. Patents 3,700,681; 3,763,173; 3,772,314 and 3,786,160. Alternative and generally improved syntheses are found in U.S Patents 3,948,919; 4,011,231; and 4,031,108; Luxembourg Patent 79564; and European Patent Applications 58069, 58070, 58071 and 58072. More recently, pirbuterol has also found utility in the treatment of congestive heart failure (U.S. Patent 4,175,128). Diazepam is a widely prescribed minor tranquilizer (The Merck Index 10th Ed., monograph no. 2967; PDR 38th Ed., pp. 1671-1674). Trimazosin (The Merck Index 10th Ed., monograph no. 9506) is an antihypertensive agent, marketed or to be marketed around the world as a hydrochloride salt, which is structurally related to prazosin.

The clinical value of the present improved formulation in inhibiting piroxicam-induced gastric irritation and ulcers is reflected by appropriate animal studies. Typical experimental protocols, in which the ability of the test compound to prevent or reduce piroxicam-induced gastric lesioning was determined, are found in the specific Examples below.

The present invention is readily carried out. The piroxicam or its salt is dosed in a mammal, particularly man, in the range of 0.1 to 1 mg/kg/day. The second medicinal agent can be dosed separately, in which case the latter will be employed in an amount within (but generally lower in) the dosage range and according to dosage regimens (frequency, routes and compositions) as specified for their alternative utility in the prior art, for example, in references cited above or further cited in said references.

Preferably and conveniently, the piroxicam and a gastric irritation and ulcer inhibiting agent of the present invention are co-administered in a single, combined formulation. This can be in a form suitable for parenteral administration, but is preferably in a form suitable for oral administration. The proportion of each drug in the combined dosage form will be in the ratio of the total daily dosage of each drug when dosed alone. The combined drugs will be dosed in single or divided doses. Single daily dosage will be most preferred in those cases where the in vivo half-life of the second medicinal agent is (like that of piroxicam) relatively long, and where the daily dosage of the second agent for a typical adult patient is relatively small, e.g., less than 1-2 grams.

In the preferred oral route of dosage, the amount of piroxicam (or salt equivalent) for an average adult patient will generally be in the range of 5-50 mg/day in combination with:

3 to 100 mg/day of pirbuterol;

2 to 40 mg/day of diazepam; or

4 to 500 mg/day of trimazosin;

an amount of the second medicinal agent generally sufficient to inhibit gastrointestinal irritation or ulcers which could otherwise be induced by the piroxicam in patients susceptible to this side effect.

The combined compounds are administered alone or in further combination with pharmaceutically-acceptable carriers or diluents. For oral use, suitable pharmaceutical carriers include inert diluents or fillers, thereby forming dosage forms such as tablets, powders, capsules, and the like. These pharmaceutical compositions can, if desired, contain additional ingredients such as flavorings, binders, excipients and the like. For example, tablets containing various excipients, such as sodium citrate, are employed, together with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials therefor include lactose or milk sugar and high molecular weight polyethylene glycols.

The present invention is illustrated by the following examples.

EXAMPLE 1

Protective Effect of Various Medicinal Agents on Piroxicam-Induced Gastric Lesions in Rats

Adult male "specific pathogen free" rats weighing 140-160 grams of the CD strain (Sprague-Dawley) were obtained from Charles River Breeding Laboratories (Kingston, N.Y.). The animals were acclimated for approximately one week and tested when they reached a body weight of 200-225 grams. The rats were fasted for 16 hours and randomized into groups consisting of 8 to 20 animals which were normalized with regard to their average body weight.

Gastric ulcers were induced in the animals by orally dosing them with a single 100 mg/kg dose of piroxicam in 2 ml. of aqueous 0.1% methylcellulose (pH = 6.8). Those animals receiving a second medicinal agent separately received the second drug in an additional 2 ml. of the same medium at about the same time. Six and one-half hours later, the animals were sacrificed by cervical dislocation and autopsied. The stomachs were surgically removed, dissected along the greater curvature and rinsed with cold water. The stomachs were individually scored for both linear and punctate lesions. The total number of lesions was used for scoring purposes. The data obtained from each group of rats was analyzed after calculation of the mean number +/- the standard error of total gastric lesions. The values obtained were also compared to the controls which received only piroxicam by the two-tailed Student's T-Test for non-paired data. The protective effect of the second medicinal agent against piroxicam-induced ulcers is shown in Table I. These data show that pirbuterol and diazepam each significantly reduce piroxicam-induced gastric lesions in the healthy fasted rat.

## TABLE I

### Protective Effect of Various Medicinal Agents on Piroxicam-Induced Gastric Lesions in Rats[a]

| Medicinal Agent[a] | Oral Dose (mg/kg) | No. of Rats in Group | Lesions/Rat ($\bar{X}$ +/- SE)[b] | Significance $p < 0.05$[c] |
|---|---|---|---|---|
| (Control) | -- | 20 | 10.1 (1.5) | |
| Pirbuterol | 3.3 | 10 | 2.8 (0.9) | + |
| | 10 | 20 | 4.5 (1.1) | + |
| | 33 | 20 | 2.6 (0.7) | + |
| (Control) | -- | 20 | 9.3 (1.3) | |
| Diazepam | 10 | 20 | 5.6 (1.1) | + |
| | 33 | 20 | 4.2 (1.1) | + |

[a]All animals, including controls, received 100 mg/kg of piroxicam.

[b]Represents the mean value $\bar{X}$ +/- the standard error (SE).

[c]As determined by the Student's two tailed T-test for non-paired data.

EXAMPLE 2

Protective Effect of Various Medicinal Agents on Gastric Lesions in Rats Induced by the Ethanolamine Salt of Piroxicam

4

According to the preceeding Example, gastric ulcers were induced by a single dose of 100 or 120 mg./kg. of the ethanolamine salt of piroxicam. Test groups received various doses of various medicinal agents as shown in Table II. These data show that the test compounds showed a significant reduction in gastric lesions induced by the ethanolamine salt of piroxicam in healthy, fasted rats.

## TABLE II

### Protective Effect of Trimazosin and Diazepam on Gastric Lesions Induced by the Ethanolamine Salt of Piroxicam

| Medicinal Agent | Oral Dose (mg/kg) | No. of Rats in Group | Lesions/Rat ($\bar{X}$ +/- SE)[b] | Significance p<0.05[c] |
|---|---|---|---|---|
| (Control)[a] | -- | 10 | 6.4 (1.1) | |
| Diazepam[a] | 10 | 10 | 5.1 (0.9) | - |
| | 33 | 10 | 1.5 (0.7) | + |
| (Control)[d] | -- | 10 | 7.6 (0.7) | |
| Trimazosin[d] | 3.3 | 10 | 5.0 (1.6) | - |
| | 10 | 10 | 3.9 (1.0) | + |
| | 33 | 10 | 5.2 (1.3) | - |

## TABLE II (Cont.)

[a] All animals, including controls, received 100 mg/kg of the ethanolamine salt of piroxicam.

[b] Represents the mean value $\bar{X}$ +/- the standard error (SE).

[c] As determined by the Student's two tailed T-test for non-paired data.

[d] All animals, including controls, received 120 mg/kg of the ethanolamine salt of piroxicam.

EXAMPLE 3

Tablets - Piroxicam (10 mg) and Pirbuterol (25 mg)

The following ingredients are combined in the following proportions by weight:

| | | |
|---|---|---|
| piroxicam ethanolamine salt (milled) | 23.68 | (equivalent to 20 of free base) |
| pirbuterol acetate | 31.28 | (equivalent to 25 of free base) |
| lactose | 220.04 | |
| hydroxypropyl methylcellulose | 4 | |
| sodium starch glycollate | 16 | |
| magnesium stearate | 5 | |

The mixture is thoroughly blended to form a uniform powder. The powder, in measured volumes corresponding to 300 mg. by weight, is compressed into tablets containing the desired potency of each active ingredient.

EXAMPLE 4

Capsules - Piroxicam (20 mg) and Pirbuterol (20 mg)

The following ingredients are combined in the following proportions by weight:

| | | |
|---|---|---|
| piroxicam (milled) | 20 | |
| pirbuterol dihydrochloride (milled) | 26.1 | (equivalent to 20 of free base) |
| cornstarch | 652.9 | |
| magnesium stearate | 3 | |

The mixture is thoroughly blended so as to obtain a uniform powder. The resultant mix (700 mg fill weight) is filled into hard gelatin capsules of a suitable size so as to obtain capsules of the desired potency.

Equivalent proportions of piroxicam ethanolamine salt (23.7) and pirbuterol monoacetate (23.4) are combined with cornstarch and magnesium stearate and filled into hard gelatin capsules in like manner to obtain capsules containing the same potency of each active ingredient.

EXAMPLE 5

Capsules - Piroxicam (20 mg) and Diazepam (10 mg)

The following ingredients are combined in the following proportion by weight:

| | |
|---|---|
| piroxicam ethanolamine salt | 23.68 (equivalent to 20 of free acid) |
| diazepam | 10 |
| calcium carbonate | 50 |
| polyethylene glycol, average molecular weight, 4000 | 166.32 |

The mixture is blended to a uniform powder and filled (250 mg fill weight) into hard gelatin capsules of a suitable size to obtain capsules of the desired potencies.

EXAMPLE 6

Tablets - Piroxicam (10 mg) and Diazepam (5 mg)

The following ingredients are combined in the following proportions by weight:

| | |
|---|---|
| piroxicam | 10 |
| diazepam | 5 |
| lactose | 123 |
| hydroxypropyl methylcellulose | 2 |
| sodium starch glycollate | 8 |
| magnesium stearate | 2 |

The mixture is thoroughly blended to form a uniform powder. Measure volumes of the powder, corresponding to 150 mg. by weight, are compressed into tablets containing the desired potency of each active ingredient.

EXAMPLE 7

Capusles - Piroxicam (20 mg) and Trimazosin (40 mg)

The following ingredients are combined in the following proportions by weight:

| | |
|---|---|
| piroxicam | 20 |
| trimazosin hydrochloride monohydrate | 45 (equivalent to 40 of free base) |
| cornstarch | 632 |
| magnesium stearate | 3 |

The mixture is thoroughly blended so as to obtain a uniform powder. The resultant mix (700 mg fill weight) is filled into hard gelatin capsules of a suitable size so as to obtain capsules of the desired potency in each drug.

**Claims**

1. An antiinflammatory composition which comprises:-

7

(a) an antiinflammatory amount of piroxicam or a pharmaceutically acceptable salt thereof; and
(b) a gastric antiirritation and ulcer-inhibiting amount of a compound selected from pirbuterol or a pharmaceutically acceptable salt thereof, (ii) diazepam and (iii) trimazosin or a pharmaceutically acceptable salt thereof.

2. An improved antiinflammatory composition which comprises:
(a) an antiinflammatory amount of piroxicam, or a pharmaceutically acceptable salt thereof; and
(b) a gastric antiirritation and ulcer-inhibiting amount of pirbuterol, or a pharmaceutically acceptable salt thereof.

3. A composition of claim 2 wherein the pirbuterol is in the form of its dihydrochloride salt and the piroxicam is in its free enolic form.

4. A composition of claims 2 wherein the pirbuterol is in the form of its monoacetate salt and the piroxicam is in the form of its ethanolamine salt.

5. An improved antiinflammatory composition which comprises:
(a) an antiinflammatory amount of piroxicam, or a pharmaceutically acceptable salt thereof; and
(b) a gastric antiirritation and ulcer-inhibiting amount of diazepam.

6. A composition of claim 5 wherein the piroxicam is in the form of its free enolic form.

7. A composition of claims 5 wherein the piroxicam is in the form of its ethanolamine salt.

8. An improved antiinflammatory composition which comprises:
(a) an antiinflammatory amount of piroxicam, or a pharmaceutically acceptable salt thereof; and
(b) a gastric antiirritation and ulcer-inhibiting amount of trimazosin, or a pharmaceutically acceptable salt thereof.

9. A composition of claim 8 wherein the trimazosin is in the form of its hydrochloride salt and piroxicam is in its free enolic form.

Claims for the following Contracting State: AT

1. A process for preparing an antiinflammatory composition which comprises combining (a) an antiinflammatory amount of piroxicam or a pharmaceutically acceptable salt thereof and (b) a gastric antiirritation and ulcer-inhibiting amount of a compound selected from pirbuterol or a pharmaceutically acceptable salt thereof (ii) diazepam and (iii) trimazosin or a pharmaceutically acceptable salt thereof.

2. A process for preparing an improved antiinflammatory composition which comprises combining:
(a) an antiinflammatory amount of piroxicam, or pharmaceutically acceptable salt thereof; and
(b) a gastric antiirritation and ulcer-inhibiting amount of pirbuterol, or a pharmaceutically acceptable salt thereof.

3. A process of claim 2 wherein the pirbuterol is in the form of its dihydrochloride salt and the piroxicam is in its free enolic form.

4. A process of claim 2 wherein the pirbuterol is in the form of its monoacetate salt and the piroxicam is in the form of its ethanolamine salt.

5. A process for preparing an improved antiinflammatory composition which comprises combining:
(a) an antiinflammatory amount of piroxicam, or a pharmaceutically acceptable salt thereof; and
(b) a gastric antiirritation and ulcer-inhibiting amount of diazepam.

6. A process of claim 5 wherein the piroxicam is in the form of its free enolic form.

7. A process of claim 5 wherein the piroxicam is in the form of its ethanolamine salt.

8. A process for preparing an improved antiinflammatory composition which comprises combining:
    (a) an antiinflammatory amount of piroxicam, or a pharmaceutically-acceptable salt thereof; and
    (b) a gastric antiirritation and ulcer-inhibiting amount of trimazosin, or a pharmaceutically acceptable salt thereof.

9. A process of claim 8 wherein the trimazosin is in the form of its hydrochloride salt and piroxicam is in its free enolic form.

**Revendications**

1. Composition anti-inflammatoire, qui comprend :
    (a) une quantité anti-inflammatoire de piroxicam ou d'un de ses sels pharmaceutiquement acceptables ; et
    (b) une quantité, inhibant l'irritation et l'ulcération gastriques, d'un composé choisi entre (i) le pirbutérol ou un de ses sels pharmaceutiquement acceptables, (ii) le diazépam et (iii) la trimazosine ou un de ses sels pharmaceutiquement acceptables.

2. Composition anti-inflammatoire améliorée, qui comprend :
    (a) une quantité anti-inflammatoire de piroxicam ou d'un de ses sels pharmaceutiquement acceptables ; et
    (b) une quantité, inhibant l'irritation et l'ulcération gastriques, de pirbutérol ou d'un de ses sels pharmaceutiquement acceptables.

3. Composition suivant la revendication 2, dans laquelle le pirbutérol est sous forme de son dichlorhydrate et le piroxicam est sous sa forme énolique libre.

4. Composition suivant la revendication 2, dans laquelle le pirbutérol est sous forme de son mono-acétate et le piroxicam est sous forme de son sel d'éthanolamine.

5. Composition anti-inflammatoire améliorée, qui comprend :
    (a) une quantité anti-inflammatoire de piroxicam ou un de ses sels pharmaceutiquement acceptables ; et
    (b) une quantité, inhibant l'irritation et l'ulcération gastriques, de diazépam.

6. Composition suivant la revendication 5, dans laquelle le piroxicam est sous sa forme énolique libre.

7. Composition suivant la revendication 5, dans laquelle le piroxicam est sous forme de son sel d'éthanolamine.

8. Composition anti-inflammatoire améliorée, qui comprend :
    (a) une quantité anti-inflammatoire de piroxicam ou d'un de ses sels pharmaceutiquement acceptables ; et
    (b) une quantité, inhibant l'irritation et l'ulcération gastriques, de trimazosine ou d'un de ses sels pharmaceutiquement acceptables.

9. Composition suivant la revendication 8, dans laquelle la trimazosine est sous forme de son chlorhydrate et le piroxicam est sous sa forme énolique libre.

Revendications pour l'Etat contractant suivant: AT

1. Procédé de préparation d'une composition anti-inflammatoire, qui consiste à mélanger (a) une quantité anti-inflammatoire de piroxicam ou d'un de ses sels pharmaceutiquement acceptables et (b) une quantité, inhibant l'irritation et l'ulcération gastriques, d'un composé choisi entre (i) le pirbutérol ou un de ses sels pharmaceutiquement acceptables, (ii) le diazépam et (iii) la trimazosine ou un de ses sels pharmaceutiquement acceptables.

2. Procédé de préparation d'une composition anti-inflammatoire améliorée, qui consiste à mélanger :
    (a) une quantité anti-inflammatoire de piroxicam ou d'un de ses sels pharmaceutiquement accepta-

9

bles ; et

(b) une quantité, inhibant l'irritation et l'ulcération gastriques, de pirbutérol ou d'un de ses sels pharmaceutiquement acceptables.

3. Procédé suivant la revendication 2, dans lequel le pirbutérol est sous forme de son dichlorhydrate et le piroxicam est sous sa forme énolique libre.

4. Procédé suivant la revendication 2, dans lequel le pirbutérol est sous forme de son mono-acétate et le piroxicam est sous forme de son sel d'éthanolamine.

5. Procédé de préparation d'une composition anti-inflammatoire améliorée, qui consiste à mélanger :

(a) une quantité anti-inflammatoire de piroxicam ou d'un de ses sels pharmaceutiquement acceptables ; et

(b) une quantité, inhibant l'irritation et l'ulcération gastrique, de diazépam.

6. Procédé suivant la revendication 5, dans lequel le piroxicam est sous sa forme énolique libre.

7. Procédé suivant la revendication 5, dans lequel le piroxicam est sous forme de son sel d'éthanolamine.

8. Procédé de préparation d'une composition anti-inflammatoire améliorée, qui consiste à mélanger :

(a) une quantité anti-inflammatoire de piroxicam ou d'un de ses sels pharmaceutiquement acceptables ; et

(b) une quantité, inhibant l'irritation et l'ulcération gastriques, de trimazosine ou d'un de ses sels pharmaceutiquement acceptables.

9. Procédé suivant la revendication 8, dans lequel la trimazosine est sous forme de son chlorhydrate et le piroxicam est sous sa forme énolique libre.

## Ansprüche

1. Entzündungshemmende Zusammensetzung, die umfaßt:

(a) eine entzündungshemmende Menge Piroxicam oder eines pharmazeutisch annehmbaren Salzes desselben und

(b) eine die Magenirritation und Ulcusbildung inhibierende Menge einer Verbindung, ausgewählt aus (i) Pirbuterol oder einem pharmazeutisch annehmbaren Salz desselben, (ii) Diazepam und (iii) Trimazosin oder einem pharmazeutisch annehmbaren Salz desselben.

2. Verbesserte entzündungshemmende Zusammensetzung, die umfaßt:

(a) eine entzündungshemmende Menge Piroxicam oder eines pharmazeutisch annehmbaren Salzes desselben und

(b) eine die Magenirritation und Ulcusbildung inhibierende Menge Pirbuterol oder eines pharmazeutisch annehmbaren Salzes desselben.

3. Zusammensetzung gemäß Anspruch 2, in welcher das Pirbuterol in Form seines Dihydrochloridsalzes vorliegt und das Piroxicam in seiner freien Enol-Form vorliegt.

4. Zusammensetzung gemäß Anspruch 2, in welcher das Pirbuterol in Form seines Monoacetatsalzes vorliegt und das Piroxicam in Form seines Ethanolaminsalzes vorliegt.

5. Verbesserte entzündungshemmende Zusammensetzung, die umfaßt:

(a) eine entzündungshemmende Menge Piroxicam oder eines pharmazeutisch annehmbaren Salzes desselben und

(b) eine die Magenirritation und Ulcusbildung inhibierende Menge Diazepam.

6. Zusammensetzung gemäß Anspruch 5, in welcher das Piroxicam in seiner freien Enol-Form vorliegt.

7. Zusammensetzung gemäß Anspruch 5, in welcher das Piroxicam in Form seines Ethanolaminsalzes vorliegt.

**8.** Verbesserte entzündungshemmende Zusammensetzung, die umfaßt:

(a) eine entzündungshemmende Menge Piroxicam oder eines pharmazeutisch annehmbaren Salzes desselben und

(b) eine die Magenirritation und Ulcusbildung inhibierende Menge Trimazosin oder eines pharmazeutisch annehmbaren Salzes desselben.

**9.** Zusammensetzung gemäß Anspruch 8, in welcher das Trimazosin in Form seines Hydrochloridsalzes vorliegt und das Piroxicam in seiner freien Enol-Form vorliegt.

Patentansprüche für folgenden Vertragsstaat: AT

**1.** Verfahren zur Herstellung einer entzündungshemmenden Zusammensetzung, das das Kombinieren:

(a) einer entzündungshemmenden Menge Piroxicam oder eines pharmazeutisch annehmbaren Salzes desselben und

(b) einer die Magenirritation und Ulcusbildung inhibierenden Menge einer Verbindung, ausgewählt aus (i) Pirbuterol oder einem pharmazeutisch annehmbaren Salz desselben, (ii) Diazepam und (iii) Trimazosin oder einem pharmazeutisch annehmbaren Salz desselben,

umfaßt.

**2.** Verfahren zur Herstellung einer verbesserten entzündungshemmenden Zusammensetzung, das das Kombinieren:

(a) einer entzündungshemmenden Menge Piroxicam oder eines pharmazeutisch annehmbaren Salzes desselben und

(b) einer die Magenirritation und Ulcusbildung inhibierenden Menge Pirbuterol oder eines pharmazeutisch annehmbaren Salzes desselben

umfaßt.

**3.** Verfahren gemäß Anspruch 2, in welchem das Pirbuterol in Form seines Dihydrochloridsalzes vorliegt und das Piroxicam in seiner freien Enol-Form vorliegt.

**4.** Verfahren gemäß Anspruch 2, in welchem das Pirbuterol in Form seines Monoacetatsalzes vorliegt und das Piroxicam in Form seines Ethanolaminsalzes vorliegt.

**5.** Verfahren zur Herstellung einer verbesserten entzündungshemmenden Zusammensetzung, das das Kombinieren:

(a) einer entzündungshemmenden Menge Piroxicam oder eines pharmazeutisch annehmbaren Salzes desselben und

(b) einer die Magenirritation und Ulcusbildung inhibierenden Menge Diazepam

umfaßt.

**6.** Verfahren gemäß Anspruch 5, in welchem das Piroxicam in seiner freien Enol-Form vorliegt.

**7.** Verfahren gemäß Anspruch 5, in welchem das Piroxicam in Form seines Ethanolaminsalzes vorliegt.

**8.** Verfahren zur Herstellung einer verbesserten entzündungshemmenden Zusammensetzung, das das Kombinieren:

(a) einer entzündungshemmenden Menge Piroxicam oder eines pharmazeutisch annehmbaren Salzes desselben und

(b) einer die Magenirritation und Ulcusbildung inhibierenden Menge Trimazosin oder eines pharmazeutisch annehmbaren Salzes desselben

umfaßt.

**9.** Verfahren gemäß Anspruch 8, in welchem das Trimazosin in Form seines Hydrochloridsalzes vorliegt und das Piroxicam in seiner freien Enol-Form vorliegt.